# EUROPEAN PATENT APPLICATION

(11) **EP 2 474 618 A1**
(43) Date of publication of application: **11.07.2012**
(21) Application number: 11150342.1
(22) Date of filing: 06.01.2011
(51) Int. Cl.: C12N 15/74

(54) **Metalloporphyrin inducible promoter**

(71) Applicant: Institut National De La Recherche Agronomique, 75007 Paris (FR); Chr. Hansen A/S, 2970 Hoersholm (DK)
(72) Inventor: Garrigues, Christel, 1963, Frederiksberg C (DK); Pedersen, Martin Bastian, 2300, København S (DK); Gaudu, Philippe, 92340, Bourg la Reine (FR); Gruss, Alexandra, 91400 Orsay (FR); Cesselin, Bénédicte, 78114, Magny Les Hameaux (FR); Lechardeur, Delphine, 78000, Versailles (FR)
(74) Representative: Vialle-Presles, Marie José

(57) **Abstract**

The invention relates to a promoter inducible by metalloporphyrins. Said promoter is useful for controlling the expression of genes of interest in gram-positive bacteria deficient in heme biosynthesis, in particular lactic acid bacteria.

## Description

The invention relates to a controlled expression system for gram-positive bacteria deficient in heme biosynthesis, in particular lactic acid bacteria.

Besides their conventional uses in the agrofoods industry, lactic acid bacteria are currently increasingly used as host cells for the production of heterologous proteins of interest. These proteins of interest can be very varied in nature, and it is therefore desirable to have as large a choice as possible of expression tools in order to be able to optimize the production thereof as a function of the specificities of each one of them.

In general, it is necessary to use strong promoters that make it possible to obtain a sufficient level of expression of the gene of interest. In certain cases, constitutive promoters can be used; in other cases (for example when the product of the gene of interest is toxic for the host bacterium or there is a risk of it interfering with the metabolism thereof), it is preferable to use inducible promoters that make it possible to initiate or to stop the expression at the desired time.

Although lactic acid bacteria have many genes whose transcription is regulated by various factors, there is currently only a relatively restricted choice of inducible promoters that can be used in practice for controlled expression of genes of interest (for review, cf. DE VOS, Curr. Op. Microbiol., 2, 289-295, 1999). Actually, this use requires not only that the promoters concerned can be regulated, but also that they have a sufficient transcriptional efficiency, and that there exists a sufficient expression differential between the various induction states; ideally, the expression should reach a high level under induction conditions and should be able to be completely blocked under non-induction conditions. In addition, it is necessary to be able to readily control the factors involved in the regulation of these promoters.

In a previous study (PEDERSEN et al., J. Bacteriol., 190, 4903-4911, 2008), the inventors' team identified in *Lactococcus lactis* a heme-responsive operon called the *ygfCBA* operon, appearing to encode a heme detoxification system. They introduced the *lacLM* reporter gene encoding a β-galactosidase, in the *L. lactis* chromosome, just downstream of the last gene of the operon, *ygfA.* They observed that when the transformed strain was grown with 100 µM heme, the *ygfCBA* operon was induced up to 50-fold when bacteria were cultivated in aeration conditions and up to 80-fold when they were cultivated in non-aeration conditions.

Studying the structure of the *L. lactis ygfCBA* operon promoter has allowed the inventors to demonstrate, besides elements similar to those conventionally present in bacterial promoters, namely the -35 (TTGACA) and -10 (TATAAT) boxes separated by 17 bp, a palindromic sequence overlapping the -35 box, which represents the YgfC-binding site.

In continuing their studies on the regulation of the *ygfCBA* operon the inventors have now found that the promoter is regulated by the product of the *ygfC* gene, YgfC. In the absence of heme, YgfC which is produced at extremely low levels, binds to the promoter of the *ygfCBA* operon and represses its transcription. When heme is added, it binds to YgfC causing its release from its binding site and allowing operon transcription.

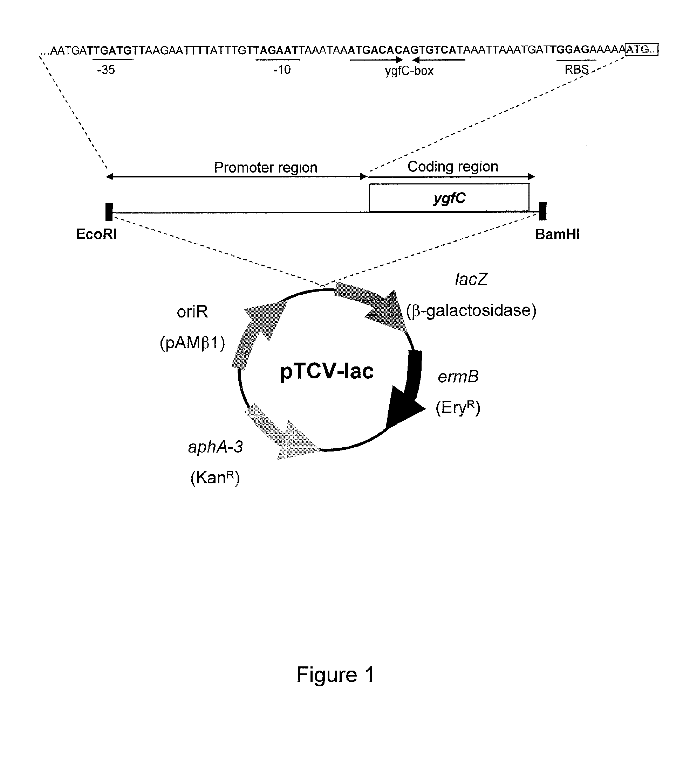

The sequence of the *L. lactis ygfCBA* operon promoter is represented on Figure 1. Besides the elements conventionally present in bacterial promoters, namely the -35 (TTGATG) and -10 (TAGAAT) boxes, it comprises a sequence located 22 nucleotides upstream of the start codon of YgfC, which represents the YgfC-binding site.

The inventors have constructed a plasmid comprising an expression system consisting of the promoter of the *ygfCBA* operon and the downstream *ygfC* gene, in fusion with the reporter gene *lacZ* (encoding beta-galactosidase) and introduced this plasmid in *Lactococcus lactis.* They have observed that in the absence of heme, the background expression of beta-galactosidase in the *Lactococcus lactis* bacteria transformed by this plasmid was essentially zero (the same as the one observed in the bacteria transformed with a plasmid carrying a promoter-less *lacZ* gene), while in the presence of heme beta-galactosidase expression increased up to 100-fold, depending on the heme concentration and the culture conditions. They further observed that, in contrast with the results reported by PEDERSEN et al. (2008, cited above), the induction was higher in aeration conditions than in non-aeration conditions.

It is known that heme, when used under aeration conditions, promotes an aerobic respiration metabolism in *L. lactis,* resulting in a stimulation of bacterial growth. The inventors have tested the effects of a heme analog, the non-iron metalloporphyrin gallium-protoporphyrin IX (Ga-PPIX), which does not promote respiration metabolism on the induction of the promoter of the *ygfCBA* operon. They observed that like heme, Ga-PPIX was an efficient inducer, both in aeration conditions and in non-aeration conditions. In contrast with heme however, it was somewhat more efficient in non-aeration conditions than in aeration conditions.

The inventors have also experimented the expression system consisting of the promoter of the *ygfCBA* operon and the downstream *ygfC* gene in *Streptococcus agalactiae,* to test whether it was functional in bacteria other than *L. lactis,* in particular in bacteria in which, unlike *L. lactis,* the combination of heme and aeration is not sufficient to promote a respiratory metabolism. They observed that both heme and Ga-PPIX were efficient inducers, active at concentration ranges even lower than in the case of *L. lactis.*

These properties of the *L. lactis ygfCBA* operon regulatory system demonstrated by the inventors make it possible to propose its use as an inducible system for the production of proteins of interest in host bacteria that do not synthesize heme, especially lactic acid bacteria.

A subject of the present invention is an expression cassette consisting of:
- a bacterial promoter, hereinafter called Pₕₑₘₑ, containing a binding site for a *Lactococcus lactis* YgfC protein, which site comprises the following sequence:
   ATGACAYAGTGTCAT (SEQ ID NO:1) wherein Y = C or T
- a sequence encoding a functional *Lactococcus lactis* YgfC protein, placed under the transcriptional control of said promoter;
- at least one cloning site allowing the insertion of a nucleotide sequence of interest under the transcriptional control of said promoter.

According to a preferred embodiment of the present invention, the Pₕₑₘₑ promoter is selected among:
- the Pₕₑₘₑ promoter of *Lactococcus lactis* subsp. *cremoris* MG1363, having the following sequence: 
AATGATTGATGTTAAGAATTTTATTTGTTAGAATTAAATAAATGACACAGTGTCATAAAT
TAAATGATTGGAGAAAAA(SEQ ID NO:2)
- the Pₕₑₘₑ promoter of *Lactococcus lactis* subsp. *cremoris* SK11 having the following sequence: 
AATTATTGAAGTTAAGAATTTTATTTGTTAGAATTAAATAAATGACATAGTGTCATAAAT
TGAATGATTGGAGAAAAAA (SEQ ID NO:3);
- the Pₕₑₘₑ promoter of *Lactococcus lactis* subsp. *lactis* IL1403 having the following sequence: 
AATAATTGATGTTAATCAATTAATTTGTTAGAATTTAATAAATGACACAGTGTCATAAAT
TGAATGAATTGGAGAAAAA (SEQ ID NO:4);
- the Pₕₑₘₑ promoter of *Lactococcus lactis* subsp. *lactis* KF147 having the following sequence: 
ATTAATTGATGTTAATCAATTAATTTGTTAGAATTTAATAAATGACACAGTGTCATAAAT
TGAATGAATTGGAGACAAAAT (SEQ ID NO: 5)
- a bacterial promoter having at least 80%, and by order of increasing preference, at least 81, 82, 83, 84, 85, 86, 87, 88, 89, 90, 91, 92, 93, 94, 95, 96, 97, 98, 99, or 100% identity, with any of the sequences SEQ ID NO: 3 to SEQ ID NO: 5.

A *Lactococcus lactis* YgfC protein is defined herein as a polypeptide having at least 80%, and by order of increasing preference, at least 81, 82, 83, 84, 85, 86, 87, 88, 89, 90, 91, 92, 93, 94, 95, 96, 97, 98, 99, or 100% identity, or having at least 90% and by order of increasing preference at least 91, 92, 93, 94, 95, 96, 97, 98, 99, or 100% similarity with the YgfC protein of *Lactococcus lactis* subsp. *cremoris* MG1363 (GenBank YP_001031969; gi:125623486). This includes, by way of non limitative examples, the YgfC protein of *Lactococcus lactis* subsp. *cremoris* SK11 (GenBank YP_808675.1; gi: 116511459), the YgfC protein of *Lactococcus lactis* subsp. *lactis* I11403 (GenBank NP_266817.1; gi: 15672643), or the YgfC protein of *Lactococcus lactis* subsp. *lactis* KF147 (GenBank YP_003353103.1; gi: 281491123).

The cloning site consists of a short sequence containing at least one restriction site allowing the insertion of DNA fragments at this location. Preferably it is a multiple cloning site, containing several restriction sites recognized by different restriction enzymes.

The present invention also encompasses expression cassettes resulting from the insertion of a heterologous nucleotide sequence of interest under the transcriptional control of the Pₕₑₘₑ promoter, into the cloning site of an expression cassette of the invention.

By "heterologous sequence" is intended a sequence other than the sequence naturally occurring downstream of the *ygfC* gene in *Lactococcus lactis.* Accordingly, said heterologous sequence does not comprise the *ygfA* and *ygfB* genes.

Said heterologous nucleotide sequence of interest may be any sequence that it is desired to express under the transcriptional control of the Pₕₑₘₑ promoter. It may in particular be any sequence encoding a heterologous protein of interest that it is desired to produce in a host lactic acid bacterium; said protein may, where appropriate, be a fusion protein, combining polypeptide sequences of diverse origin.

The present invention also encompasses recombinant vectors containing an expression cassette of the invention, as well as host gram-positive bacteria deficient in heme biosythesis, in particular lactic acid bacteria, transformed with a recombinant vector of the invention.

Preferably, said host bacteria are selected among *Lactococci, Streptococci, Lactobacilli, Leuconostoc,* or *Enterococci.* Examples of particularly preferred host bacteria are those belonging to the species *Lactococcus lactis* (subsp. *lactis* or *cremoris), Streptococcus agalactiae,* or *Streptococcus thermophilus.*

A subject of the present invention is also the use of expression cassettes of the invention and of recombinant vectors containing said expression cassettes for producing proteins of interest in lactic acid bacteria.

In particular, the present invention provides a method for producing a protein of interest in lactic acid bacteria, characterized in that it comprises:
- introducing into said bacteria an expression cassette of the invention, comprising a sequence encoding said protein of interest;
- culturing said bacteria in a medium suitable for its growth, to which is added an amount of metalloporphyrin that is sufficient to induce the expression of said protein;
- recovering the protein produced.

The culture medium can be any nutrient medium that is suitable for growing the strain(s) or species of lactic acid bacteria concerned, provided that it does not include significant levels of heme or any other metal protoporphyrin. Classically, it contains a carbon source, in the form of sugar(s) that can be assimilated, a nitrogen source, generally in the form of a mixture of amino acids, as well as mineral salts and vitamins. Non-limitative examples of suitable growth media include M17, MRS, BHI, or THY, which are common media for lactic acid bacteria growth.

The metalloporphyrin is preferably selected among heme and gallium-protoporphyrin IX (Ga-PPIX).

The metalloporphyrin can be added to the culture medium prior to inoculation of the bacteria, or simultaneously with the bacterial inoculum. It can also be added at any time during growth of the culture, preferably when the bacteria exit the lag phase and start the exponential phase.

According to a preferred embodiment of the method of invention, the bacteria are cultivated under non-aeration conditions (i.e as nonagitated cultures in full bottles, or as agitated or non-agitated cultures in anaerobiosis, for example in an anaerobic chamber in which the ambient gas typically comprises 5% H₂, 5% CO₂, and 90% N₂)

According to another preferred embodiment of the present invention, the bacteria are cultivated under aeration. Aeration can be effected by any means known per one skilled in the Art, for example by shaking or stirring the culture under an atmosphere such as air, containing oxygen (typically in flasks 1/10^{th} filled with culture and shaken at 150-250 revolutions per minute, depending on the aerobic tolerance of the bacterium), or by passing air or another gaseous mixture containing oxygen into the culture medium.

Generally, the protein of interest is recovered when the cultures are in late exponential phase or in stationary phase, if the metalloporphyrin has been added to the culture medium prior to or simultaneously with the bacterial inoculum. If the metalloporphyrin has been added to the culture medium during the culture, the protein of interest is preferably recovered between one and two hours after this addition.

When heme is used as the inducer, cultivation under aeration generally allows one to obtain higher levels of induction than cultivation under non-aeration conditions. In the case of host bacteria which are able to establish a respiration metabolism, such as *L. lactis,* cultivation under aeration combined with the use of heme also results in the stimulation of bacterial growth and thus a better biomass yield, and in a higher pH of the culture medium which can be advantageous to limit denaturation of low pH-sensitive proteins.

When a non-iron metalloporphyrin, such as Ga-PPIX is used as the inducer, cultivation under non-aeration conditions generally allows one to obtain as good or higher levels of induction than cultivation under aeration. No growth stimulation of the host bacteria occurs, which might allow the cell to channel energy to protein production. Also, non-aeration conditions may be useful for production of oxygen-sensitive recombinant proteins. In this case, cultures can be buffered to avoid protein denaturation due to medium acidification.

The amount of inducer in the culture medium is generally from 1 to 50 µM. However, the optimal concentrations may vary according to the strains and species being used as host bacteria (due to variable heme uptake). By way of example, preferred concentrations of hemin or Ga-PPIX for *Lactococcus lactis* are generally from 10 to 30 µM, preferably about 20 µM. In the case of *Streptococcus agalactiae,* optimal concentrations are 10 fold lower; cultivation under aeration conditions with 1 µM heme was preferable to 10 µM under non-aeration or aeration conditions in M17 or in BHI-Tris.

Based on the information provided herein by the description of the present invention, those skilled in the art can readily determine, by means of prior tests carried out, for example, by placing a reporter gene under the control of the Pₕₑₘₑ/YgfC system in an expression cassette of the invention, the most suitable amounts of the inducer according to the bacterial species or strain concerned, the operating conditions used (such as the medium used, the nature of the inducer and the schedule for adding it), and the desired level of expression.

The present invention will be understood more thoroughly from the additional description which follows, which refers to non-limiting examples illustrating the construction of an expression cassette in accordance with the invention and its use in different species of host bacteria.

### EXAMPLE 1: CONSTRUCTION OF AN EXPRESSION VECTOR CONTAINING THE lacZ REPORTER GENE UNDER THE CONTROL OF THE ygfCBA OPERON REGULATORY SYSTEM

A DNA fragment of 753 nucleotides is amplified by PCR with the primer pairs:
Primer 1: GATCGAATTCGCTGATTAATATCTGTCAG (SEQ ID NO:6);
Primer 2: GATCGGATCCTATTTTTTGCTTTCAGTAAC (SEQ ID NO:7).
and genomic DNA of *L. lactis* MG1363 as a template.

The amplified DNA fragment, covering the *ygfCBA* promoter (Pₕₑₘₑ) preceded by a transcriptional terminator and followed by the *ygfC* open reading frame, is digested by EcoRI and BamHI, and then cloned into the EcoRI-BamHI sites of pTCV-lac plasmid (POYART & TRIEU-CUOT, FEMS Microbiology Letters, 156, 193-8, 1997) upstream of the plasmid-carried *lacZ* gene.

Figure 1 schematises the structure of the pTCV-lac plasmid and of the insert representing the *Pₕₑₘₑ-ygfC* system. The sequence of the Pₕₑₘₑ promoter (SEQ ID N0: 2) followed by the start codon of YgfC (in bold characters and boxed) is also represented. The potential -35 and -10 boxes and the ygfC-box of the promoter and the potential RBS (ribosome binding site) of *ygfC* are represented in bold characters and underlined.

### EXAMPLE 2: INDUCTION OF THE EXPRESSION OF THE lacZ REPORTER GENE IN LACTOCOCCUS LACTIS.

The pTCV-lac plasmid was used to transform the *L. lactis* strain MG1363. The transformants were selected on M17 plus glucose (0.5%) solid medium containing erythromycin 3 µg/ml. Colonies were purified and cells stored at -80°C.

### Preparation of the inducers:

Hemin solution is prepared at 20 mM in 50 mM NaOH, and aliquoted for storage at -20°C; one tube is used for each assay and is then discarded.

PPIX-Ga solution is prepared at 10 mM in dimethylsulfoxide, and prepared in aliquots for storage at - 20°C; one tube is used for each assay and is then discarded.

### Bacterial cultures

The *L. lactis* cells carrying the plasmid are precultured overnight at 30°C under non-aeration conditions in M17-glucose (0.5%) medium or BHI-Tris (note that BHI includes 0.2% glucose). The media are supplemented with 3 µg/ml erythromycin to maintain the plasmid.

The following day, the precultures are diluted 100-fold in new M17-glucose (1%) medium or BHI-Tris medium (0.5% final glucose concentration), and containing 3 µg/ml erythromycin. The cultures are grown at 30°C either under non-aeration conditions (nonagitated cultures in full bottles), or under aeration conditions (cultures in conical flasks with baffles, one tenth full; agitation at 170 rpm in the presence of air). The growth is monitored by measuring the OD₆₀₀.

The inducer is added either simultaneously with the bacteria, or during growth when the bacteria exit the lag phase (OD₆₀₀=∼0.1 in M17-glucose [1%] medium, OD₆₀₀=~0.2 to 0.3 in BHI-Tris-glucose medium).

### Assay of the beta-galactosidase activity.

Cells are recovered from the cultures at OD₆₀₀=1, and beta-galactosidase activity of the bacterial extracts is quantified by the Miller method (MILLER, Experiments in molecular genetics. Cold Spring Harbor Laboratory Press, Cold Spring Harbor, NY, 1972). In the presence of o-nitrophenyl-β-D-galactoside (ONPG), beta-galactosidase produces yellow-colored *O*-nitrophenol which can be assayed by measuring the optical density at 420 nm (A₄₂₀) ; Beta-galactosidase activity of each sample is measured in Miller units by the following formula: 1000×A420/T (min) × V (ml) x A₆₀₀. This formula measures activity in a cell sample, such that the value is standardized to the same cell biomass (given as the A₆₀₀ value).

### Results:

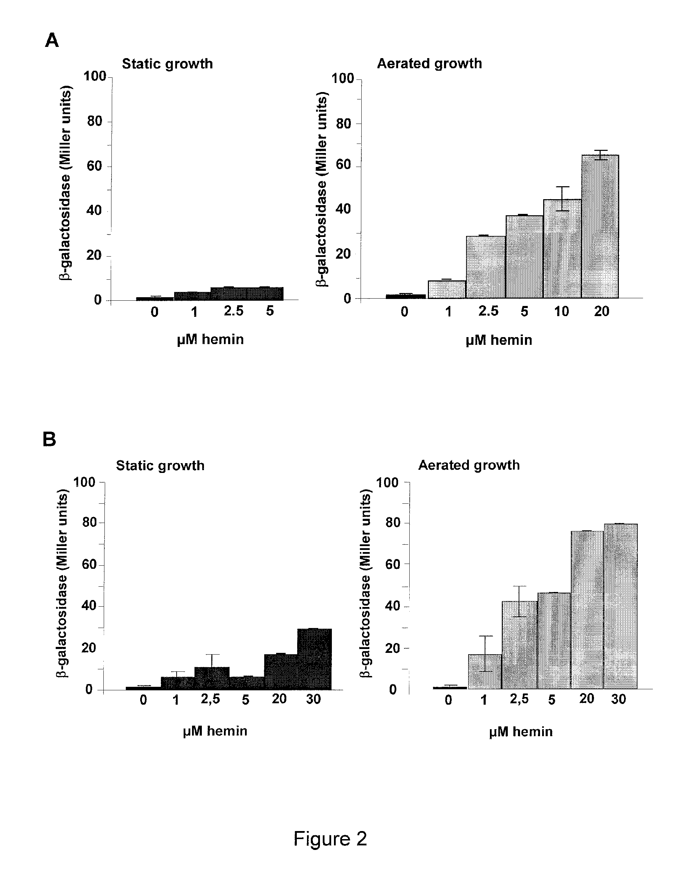

Figure 2 shows the induction under non-aerated (static) or aerated growth conditions in M17-glucose (1%) medium by different concentrations of hemin added simultaneously with the inoculum (A) or added at OD₆₀₀=∼0.1 (B). The cells were recovered at OD₆₀₀=1.

These results show that induction is far more efficient when hemin is added to cells grown under shaking aeration conditions than to cells grown under non-aeration conditions. Efficiency is about the same when hemin is added during growth and when it is added simultaneously with the bacteria (respectively 80- and 70-fold induction).

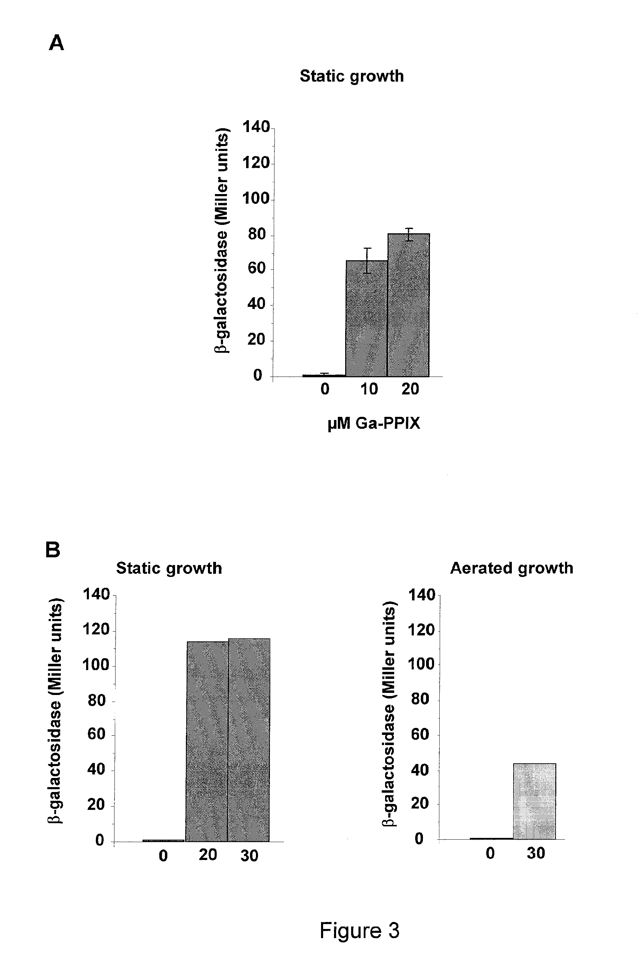

Figure 3 shows the induction under non-aerated (static) growth conditions in M17-glucose (1%) medium by 10 or 20 µM of Ga-PPIX added simultaneously with the inoculum (A), or the induction under non-aerated (static) or aerated growth conditions in M17-glucose (1%) medium by 10 or 20 µM of Ga-PPIX added at OD₆₀₀∼0.1 (B). Cells were recovered at OD₆₀₀=1 .

These results indicate that induction is more efficient when Ga-PPIX is added to cells grown under non-aeration (static) conditions than to cells grown under aeration conditions. The fold induction was similar when Ga-PPIX is added during growth and when it is added simultaneously with the bacteria (respectively 120-fold and 90-fold).

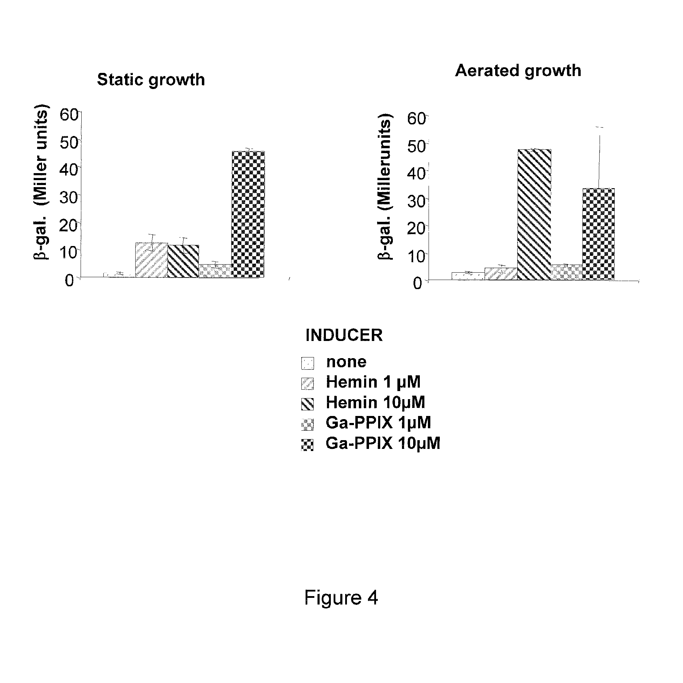

Figure 4 shows the induction under non-aerated (static) or aerated growth conditions in BHI-Tris-glucose medium by different concentrations of hemin or Ga-PPIX added at OD₆₀₀=∼0.2 to 0.3. The cells were recovered after 1 hour of treatment (corresponding to the following final OD₆₀₀: for non-aerated growth, control(no hemin) =1 ; 1 µM hemin=1; 10 µM hemin=0.6. For aerated growth, control=0.7; 1 µM hemin=0.7; 10 µM hemin=0.7. For experiments with Ga-PPIX, non-aerated growth, control=1; 1 µM Ga-PPIX=1; 10 µM Ga-PPIX=1. For aerated growth, control=0.7; 1 µM GaPPIX=0.7; 10 µM Ga-PPIX=0.7).

These results confirm that the induction by Ga-PPIX is at least as efficient when cells are grown under non-aeration conditions as when they are grown under aerated conditions and that the induction by hemin is generally more efficient when cells are grown under aerated conditions than when they are grown under non-aeration conditions.

In all the culture conditions tested, the background level of beta-galactosidase activity in absence of inducer is very low.

### EXAMPLE 3: INDUCTION OF THE EXPRESSION OF THE lacZ REPORTER GENE IN STREPTOCOCCUS AGALACTIAE

The pTCV-lac plasmid was used to transform the *Streptococcus agalactiae* strain NEM316. The transformants were selected on solid M17-glucose (0.5%) medium containing erythromycin 3µg/ml.

### Preparation of the inducers:

Hemin and PPIX-Ga solutions are prepared as described in Example 2 above.

### Bacterial cultures

The *Streptococcus agalactiae* cells carrying the plasmid are precultured overnight at 37°C in M17-glucose (0.2%) and 3 µg/ml erythromycin, or in BHI-Tris medium supplemented with 3 µg/ml erythromycin.

The following day, the precultures are diluted 100-fold in new M17-glucose (1%) medium supplemented with 3 µg/ml erythromycin, or BHI-Tris-glucose (0.5% final concentration), and 3 µg/ml erythromycin. The cultures are grown at 37°C either under non-aeration conditions or under aeration conditions and growth is monitored by measuring the OD₆ₒₒ as described above for *Lactococcus lactis.*

The inducer is added during growth when bacteria exit the lag phase (OD₆₀₀=∼0.25 to 0.3) .

### Assay of beta-galactosidase activity.

Cells are recovered from the cultures at OD₆₀₀=1, and bacterial beta-galactosidase activity is quantified as described in Example 2 above.

### Results:

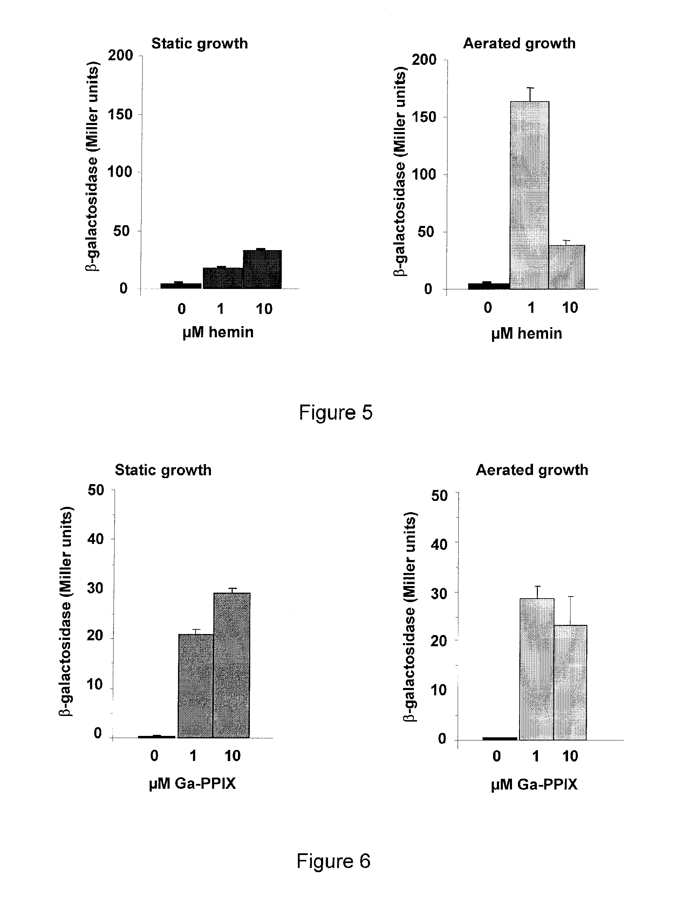

Figure 5 shows the induction under non-aerated (static) or aerated growth conditions in M17-glucose medium by 1 or 10 µM of hemin added at OD₆₀₀∼0.3. The cells are recovered after one hour of treatment (final OD₆ₒₒ: for non-aerated growth, control(no hemin) =0.7; 1 µM hemin=0.8; 10 µM hemin=0.8. For aerated growth, control=0.7; 1 µM hemin=0.6; 10 µM hemin=0.5.).

These results show that, like in *Lactococcus lactis,* the induction is greater when hemin is added to cells grown under aeration conditions (more than 150-fold over background without hemin) than to cells grown under non-aeration conditions(less than 50-fold). The amount of hemin resulting in optimal induction (1 µM) is 10 to 20 times lower than in the case of *Lactococcus lactis.*

Figure 6 shows the induction under non-aerated (static) or aerated growth conditions in M17-glucose medium, by 1 or 10 µM of Ga-PPIX added at OD₆₀₀∼0.3. The cells are recovered after one hour of treatment (final OD₆ₒₒ: for non-aerated growth, control(no Ga-PPIX)=0.8; 1 µM Ga-PPIX=0.8; 10 µM Ga-PPIX=0.8. For aerated growth, control=1; 1 µM GaPPIX=0.8; 10 µM Ga-PPIX=0.8.).

These results show that the efficiency of induction is similar when Ga-PPIX is added to *Streptococcus agalactiae* cells grown under non-aeration conditions or aeration conditions. Optimal induction is obtained for 1µM to 10 µM under both non-aeration and aeration conditions.

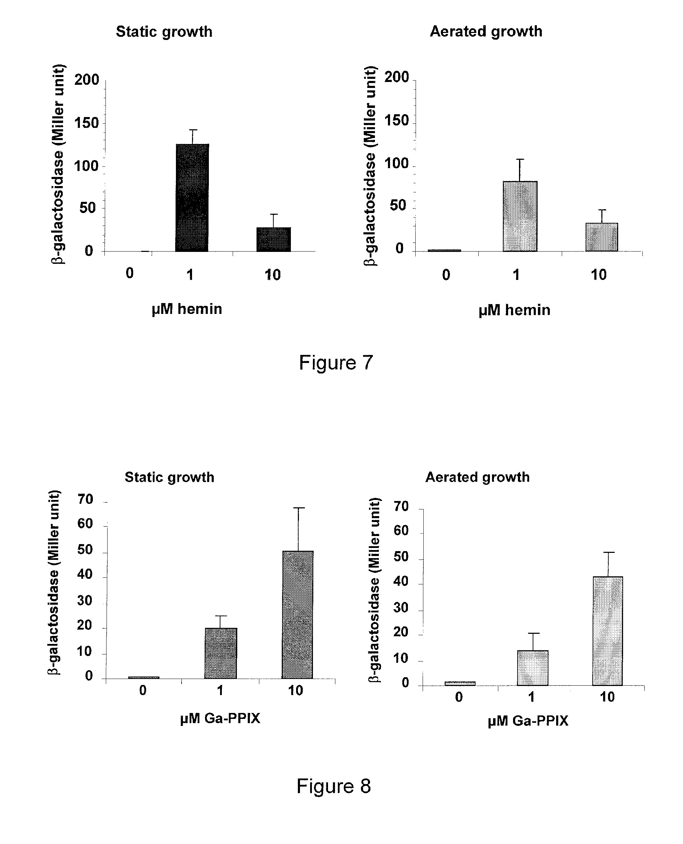

Figure 7 shows the induction under non-aerated (static) or aerated growth conditions in BHI-Tris-glucose medium by 1 or 10 µM of hemin added at OD₆₀₀∼0.25. The cells are recovered after one hour of treatment (final OD₆ₒₒ: for non-aerated growth, control(no hemin)=0.4; 1 µM hemin=0.4; 10 µM hemin=0.4. For aerated growth, control=0.5; 1 µM hemin=0.4; 10 µM hemin=0.4).

In these conditions the induction by hemin is efficient in both non-aerated and aerated cultures. It appears somewhat more efficient when cells are grown under non-aeration conditions than under aerated conditions. In either non-aeration or aerated conditions optimal induction is obtained for 1 µM hemin.

Figure 8 shows the induction under non-aerated (static) or aerated growth conditions in BHI-Tris-glucose medium by 1 or 10 µM of Ga-PPIX added at OD₆₀₀∼0.25. The cells are recovered after one hour of treatment (final OD₆ₒₒ: for non-aerated growth (no Ga-PPIX), control=0.4; 1 µM Ga-PPIX=0.4; 10 µM Ga-PPIX=0.4. For aerated growth, control=0.5; 1 µM GaPPIX=0.5; 10 µM Ga-PPIX=0.5) .

Induction by Ga-PPIX is efficient when cells are grown under non-aeration conditions as well as when they are grown under aeration conditions. The level of induction appears a little higher under non-aeration conditions. In either non-aeration or aeration conditions optimal induction is obtained for 10 µM Ga-PPIX .

Like in *Lactococcus lactis,* the background level of beta-galactosidase activity in *Streptococcus agalactiae* in absence of inducer is very low for all the culture conditions tested.

## Claims

1. An expression cassette consisting of:
- a bacterial promoter, herein called Pₕₑₘₑ, containing a binding site for a *Lactococcus lactis* YgfC protein, comprising the following sequence:
ATGACAYAGTGTCAT (SEQ ID N0:1) wherein Y = C or T,
- a sequence encoding a functional *Lactococcus lactis* YgfC protein, placed under the transcriptional control of said promoter;
- at least one cloning site allowing the insertion of a nucleotide sequence of interest under the transcriptional control of said promoter.

2. The expression cassette as claimed in claim 1, **characterized in that** the Pₕₑₘₑ promoter is selected among:
- the Pₕₑₘₑ promoter of *Lactococcus lactis* subsp. *cremoris* MG1363, having the following sequence(SEQ ID NO:2) : 
AATGATTGATGTTAAGAATTTTATTTGTTAGAATTAAATAAATGACACAGTGTCATAAAT
TAAATGATTGGAGAAAAA
- the Pₕₑₘₑ promoter of *Lactococcus lactis* subsp. *cremoris* SK11 having the following sequence (SEQ ID NO:3): 
AATTATTGAAGTTAAGAATTTTATTTGTTAGAATTAAATAAATGACATAGTGTCATAAAT
TGAATGATTGGAGAAAAA;
- the Pₕₑₘₑ promoter of *Lactococcus lactis* subsp. *lactis* IL1403 having the following sequence (SEQ ID NO:4): 
AATAATTGATGTTAATCAATTAATTTGTTAGAATTTAATAAATGACACAGTGTCATAAAT
TGAATGAATTGGAGAAAAA;
- the Pₕₑₘₑ promoter of Lactococcus lactis subsp. lactis KF147 having the following sequence (SEQ ID NO: 5): 
ATTAATTGATGTTAATCAATTAATTTGTTAGAATTTAATAAATGACACAGTGTCATAAAT
TGAATGAATTGGAGACAAAAT
- a bacterial promoter having at least 80% identity, with any of the sequences SEQ ID NO: 3 to SEQ ID NO: 5.

3. An expression cassette resulting from the insertion of an heterologous nucleotide sequence of interest under the transcriptional control of the Pₕₑₘₑ promoter, into the cloning site of an expression cassette of any one of claims 1 or 2.

4. A recombinant vector comprising an insert consisting of an expression cassette of any one of claims 1 to 3.

5. A gram-positive bacterium deficient in heme biosynthesis, transformed with a recombinant vector of claim 4.

6. A transformed gram-positive bacterium of claim 5, which is a lactic acid bacterium.

7. A transformed lactic acid bacterium of claim 6, selected among *Lactococci, Streptococci, Lactobacilli, Leuconostoc,* or *Enterococci.*

8. A transformed lactic acid bacterium of claim 7, selected among *Lactococcus lactis, Streptococcus agalactiae,* or *Streptococcus thermophilus.*

9. A method for producing a protein of interest in a gram-positive bacterium, **characterized in that** it comprises:
- providing a transformed gram-positive bacterium of any of claims 5 to 8 containing an expression cassette of claim 3 comprising a sequence encoding said protein of interest under transcriptional control of the Pₕₑₘₑ promoter;
- culturing said bacteria in a medium containing an amount of metalloporphyrin that is sufficient to induce the expression of said protein of interest;
- recovering the protein produced.

10. The method of claim 9, wherein the metalloporphyrin is selected among heme and gallium-protoporphyrin IX (Ga-PPIX).

11. The method of any of claims 9 or 10, wherein the lactic acid bacterium is cultivated under non-aeration conditions.

12. The method of claim 11, wherein the metalloporphyrin is Ga-PPIX

13. The method of any of claims 9 or 10, wherein the lactic acid bacterium is cultivated under aeration conditions.

14. The method of claim 13, wherein the metalloporphyrin is heme.

15. The method of any of claims 9 to 14, wherein the amount of metalloporphyrin in the culture medium is from 0.5 to 50 µM.


SEQUENCE LISTING

<110> INSTITUT NATIONAL DE LA RECHERCHE AGRONOMIQUE
CHR HANSEN A/S

<120> METALLOPORPHYRIN INDUCIBLE PROMOTER

<130> MJP/11-F539-150EP

<160> 7

<170> PatentIn version 3.5

<210> 1
<211> 15
<212> DNA
<213> Lactococcus lactis

<400> 1
atgacayagt gtcat 15

<210> 2
<211> 78
<212> DNA
<213> Lactococcus lactis subsp. cremoris MG1363

<400> 2
aatgattgat gttaagaatt ttatttgtta gaattaaata aatgacacag tgtcataaat 60

taaatgattg gagaaaaa 78

<210> 3
<211> 78
<212> DNA
<213> Lactococcus lactis subsp. cremoris SK11

<400> 3
aattattgaa gttaagaatt ttatttgtta gaattaaata aatgacatag tgtcataaat 60

tgaatgattg gagaaaaa 78

<210> 4
<211> 79
<212> DNA
<213> Lactococcus lactis subsp. lactis IL1403

<400> 4
aataattgat gttaatcaat taatttgtta gaatttaata aatgacacag tgtcataaat 60

tgaatgaatt ggagaaaaa 79

<210> 5
<211> 81
<212> DNA
<213> Lactococcus lactis subsp. lactis KF147

<400> 5
attaattgat gttaatcaat taatttgtta gaatttaata aatgacacag tgtcataaat 60

tgaatgaatt ggagacaaaa t 81

<210> 6
<211> 29

<212>  DNA
<213>  Artificial

<220>
<223>  PCR primer

<400>  6
gatcgaattc gctgattaat atctgtcag                                          29


<210>  7
<211>  30
<212>  DNA
<213>  Artificial

<220>
<223>  PCR primer

<400>  7
gatcggatcc tattttttgc tttcagtaac                                          30